# EUROPÄISCHE PATENTSCHRIFT

(11) **EP 3 321 343 B1**
(45) Veröffentlichungstag und Bekanntmachung des Hinweises auf die Patenterteilung: **20.10.2021**
(21) Anmeldenummer: 17202455.6
(22) Anmeldetag: 05.08.2011
(51) Int. Cl.: C09K 21/14, C08G 79/02, C08K 13/02

(54) **FLAMMSCHUTZMITTELZUSAMMENSETZUNGEN ENTHALTEND TRIAZIN-INTERKALIERTE METALL-PHOSPHATE**
FLAME PROTECTION AGENT COMPOSITIONS CONTAINING TRIAZINE INTERCALATED METAL PHOSPHATES
COMPOSITIONS IGNIFUGEANTES CONTENANT DES PHOSPHATES MÉTALLIQUES INTERCALÉS DE TRIAZINE

(30) Priorität: 23.08.2010 DE 102010035103
(43) Veröffentlichungstag der Anmeldung: 16.05.2018
(62) Teilanmeldung aus: 11748333.9
(73) Patentinhaber: J.M. Huber Corporation, Atlanta, GA 30339 (US)
(72) Erfinder: KÖSTLER, Hans-Günter, 64646 Heppenheim (DE); DAVE, Trupti, 64673 Zwingenberg (DE); WEHNER, Wolfgang, 64673 Zwingenberg (DE)
(74) Vertreter: Murgitroyd & Company

(56) Entgegenhaltungen:
- WO-A2-02/48248
- DE-A1-102007 036 465
- HIROKAZU NAKAYAMA: "INTERCALATION OF ORGANIC MOLELECULES INTO LAYERED PHOSPHATES", PHOSPHORUS RESEARCH BULLETIN, Bd. 23, 1. Januar 2009 (2009-01-01), Seiten 1-9, XP055017083, ISSN: 0918-4783, DOI: 10.3363/prb.23.1
- JENNY ALONGI ET AL: "Flame retardancy properties of [alpha]-zirconium phosphate based composites", POLYMER DEGRADATION AND STABILITY, Bd. 95, Nr. 9, 27. April 2010 (2010-04-27) , Seiten 1928-1933, XP055017029, ISSN: 0141-3910, DOI: 10.1016/j.polymdegradstab.2010.04.007
- "Melamine derivatives", SpecialChem S.A. , 28. Juni 2009 (2009-06-28), XP002667710, Gefunden im Internet: URL:http://web.archive.org/web/20090628141 703/http://www.specialchem4polymers.com/tc /Melamine-Flame-Retardants/index.aspx?id=4 004 [gefunden am 2012-01-20]

## Beschreibung

Die vorliegende Erfindung betrifft Flammschutzmittelzusammensetzungen enthaltend Triazin-interkalierte Metall-Phosphate mit offenen Gerüststrukturen (Open Framework), deren Verwendung, solche Metallphosphate sowie deren Herstellung.

Es ist bekannt, organophile Schichtsilikate, welche beispielsweise mittels Ionenaustausch hergestellt wurden, als Füllmaterialien für thermoplastische Werkstoffe sowie für Duroplaste zu verwenden, wobei Nanocomposite erhalten werden. Bei der Verwendung von geeigneten organophilen Schichtsilikaten als Füllmaterialien werden die physikalischen und mechanischen Eigenschaften der derart hergestellten Formteile erheblich verbessert. Von besonderem Interesse ist die Erhöhung der Steifigkeit bei mindestens gleichbleibender Zähigkeit. Besonders gute Eigenschaften zeigen Nanocomposite, welche das Schichtsilikat in exfoliierter Form enthalten. Diese Nanocomposite werden bevorzugt als Flammschutzmittel bzw. als Synergisten eingesetzt.

Aus WO-A 00/44669 sind organophile Schichtsilikate bekannt, die durch Behandlung eines natürlichen oder synthetischen Schichtsilikats oder eines Gemisches solcher Silikate, mit einem Salz einer - gegebenenfalls quaternären - cyclischen Melaminverbindung oder einem Gemisch solcher Salze, hergestellt werden.

Ähnliche Überlegungen sollten auch für organophile Metall-Phosphate mit offenen Gerüststrukturen gelten (s. hierzu Def. "A Review of Open Framework Structures", Annu. Rev. Mater. Sci. 1996, 26, 135-151), insbesondere für jene, die mit Melamin interkaliert sind (Interkalat auch Einlagerungsverbindung genannt, s. hierzu Def. in RÖMPP, Chemielexikon, 9.Aufl., 1995 ,G. Thieme, Bd. 3, S2005).

In der Literatur sind diverse Melamin-Phosphate beschrieben, die keine offenen Gerüststrukturen aufweisen. So Melamin-ortho-Phosphat in Magn. Reson. Chem. 2007, 45, S231-S246., Bis-Melamin-di(pyro)-Phosphat in J. Phys. Chem. B 2004, 108, 15069-15076 und Melamin-poly-Phosphat in J. Phys. Chem. B 2005, 109, 13529-13537. Deren Verwendung als Flammschutzmittel ist dort in der zitierten Sekundärliteratur aufgeführt.

Gewisse Melamin-Metall-Phosphate sind beschrieben in WO-A 2009/015772. Diese Verbindungen besitzen aber - wie an der Aluminiumverbindung gezeigt - nur eine begrenzte (Thermo)Eigenstabilität, die für eine Einarbeitung in Polyamiden nicht ausreichend ist (siehe Beispiele 7 und 8).

Melamin-interkalierte Zirkonium-(Schicht)-Phosphate sind bekannt aus Solid State Sciences 2009, 11, 1007-1015. Die Verwendung als Kunststoffadditive, insbesondere als Flammschutzmittel ist hierin aber nicht beschrieben. Andere Melamin-interkalierte (Metall)Schichtphosphate sind in der Literatur nicht belegt.

Eine Interkalation von α, ω-Alkandiaminen in Aluminium-(Schicht) Triphosphat ist publiziert in J. Inclusion Phenomena andMacrocyclic Chem. 1999, 34 401-412.

Die Schichtstruktur von Aluminiumtriphosphat wird in Chem. Commun. 2006, 747-749 belegt. Eine offene Netzstruktur ist für Ethylendiamin-Zinkphosphat-Addukte aus Zeolites and Related Microporous Materials 1994, 2229-2236 bekannt.

Ethylendiamin-bis-zinkphosphat ist in US 5994435 und US6207735 als Flammschutzmittel beansprucht. In JP 8269230 sind Amin-Zinkphosphat beschrieben, die auch die Anionen HPO₄, H₂PO₄, Zn₂(HPO₄)₃ und Zn₄[(PO₄)₂ (HPO₄)₂] umfassen. Die Anmeldungen JP9040686, JP10259275, JP11152373, JP11199708, JP11246754, JP11269187, JP11293155, JP2000063562, JP2000063563, JP2000154283, JP2000154287, JP2000154324 und JP2001031408 beschreiben Verfahren zur Herstellung spezieller Anwendungsformen sowie Kombinationen von Ethylendiamin-Zinkphosphat. Die Verfahren sind jedoch unwirtschaftlich, da sie entweder mit H₃PO₄-Überschuss arbeiten oder von Zn(en)₃-Komplexen ausgehen. JP9169784 und JP2001011462 publizieren Diethylentriamin- bzw. Piperazin-Zinkphosphat-Komplexe als Flammschutzmittel.

Anorganische Phosphate mit offenen Gerüststrukturen sind in einem Aufsatz in Angew. Chem. 1999, 111, 3466-3492 beschrieben.

Nachteilig an den genannten Verbindungen aus dem Stand der Technik sind die begrenzte (Thermo)-Eigenstabilität und die nach Einarbeitung ins Polymersubstrat resultierenden ungünstigen mechanischen Eigenschaften.

Die Aufgabe besteht darin, Flammschutzmittelzusammensetzungen bereitzustellen, die ein hohes Maß an (Thermo)-Eigenstabilität aufweisen und dem Polymeren nach Einarbeitung hervorragende mechanische Eigenschaften verleihen.

Die Aufgabe wurde unter anderem gelöst durch die Bereitstellung von Flammschutzmittelzusammensetzungen enthaltend
mindestens ein Triazin-interkaliertes Metall-Phosphat mit mindestens einer Monomereinheit der folgenden allgemeinen Formel (I)

(A - H)ₐ⁽⁺⁾[M_{b}^{m+}(H₂PO₄)ₓ₁⁽⁻⁾(HPO₄)ₓ₂²⁽⁻⁾(PO₄)ₓ₃³⁽⁻⁾(PO₃)_{y}⁽⁻⁾]^{(a-)}∗pH₂O (I)

wobei
(A - H)⁽⁺⁾ ein Triazin-Derivat der Formeln (II-1), (II-2) oder (II-3) 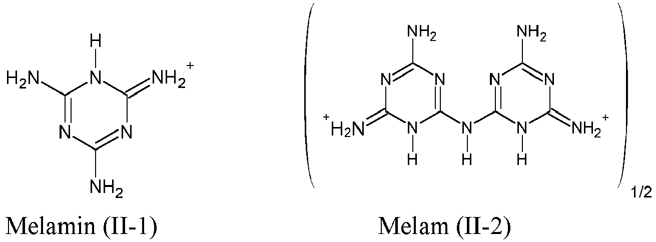 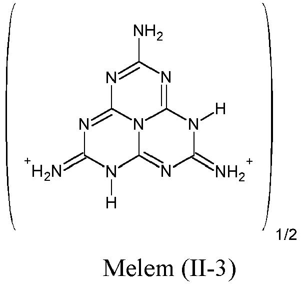 ist; jedes **M** unabhängig Mg, Zn oder Al ist;
**a** 1 bis 6,
**b** 1 bis 14,
**m=** 1 bis 4,
**x₁, x₂, x₃, y** = 0 bis 12, wobei mindestens eine der Variablen x₁, x₂, x₃ > 0 und p = 0 bis 5 ist,
wobei gilt: a + mb = x₁ + 2x₂ + 3x₃ + y; und wobei:
a) das Monomer Folgendes beinhaltet: (i) einen gemischten Typ, zusammengesetzt aus Orthophosphat und Pyrophosphatliganden; (ii) einen gemischten Typ, zusammengesetzt Metaphosphat und Pyrophosphatliganden; oder (iii) Triphosphatliganden beinhaltet; oder
b) wobei x₁ > 0.

In einer bevorzugten Ausführungsform der Erfindung weisen die Flammschutzmittelzusammensetzungen enthaltend die Triazin-interkalierten Metall-Phosphate der Formel (I) offene Gerüststrukturen auf. Die Triazinderivate sind ebenso wie Melon als chemische Vorstufen für Kohlenstoff-Nitrid (C₃N₄)ₓ bekannt.

Triazin-interkalierte Metall-Phosphate, insbesondere mit offenen Gerüststrukturen, welche durch direkte Umsetzung von (wässrigen) aciden Metall-Phosphaten mit Melamin und optionaler nachfolgender Temperung aus den entsprechenden (Precursor) Vorläuferstufen hergestellt werden, zeigen eine hohe Thermostabilität bei der Verarbeitung kombiniert mit ausgezeichneter Dispergierwirkung und Grenzflächenadhäsion. Diese Systeme zeichnen sich durch überraschend gute Schichtseparation aus, verbunden mit ausgezeichneter Adhäsion zu einer Vielzahl von Polymeren und Füllstoffen. Weiterhin überraschend ist, dass die erfindungsgemäßen Triazin-interkalierten Metall-Phosphate mit offenen Gerüststrukturen nicht nur hervorragende Füllstoffe zur Verbesserung der mechanischen Eigenschaften von Polymeren sind, sondern auch als Flammschutzmittel wirken. Die Triazin-interkalierten (Metall)Phosphate mit offenen Gerüststrukturen können auch aus Ketten(Band)-Phosphaten (Catena-Typ), Blattphosphaten (Leiter- oder Phyllo-Typ - alle mit 1-D-Strukturen), Schichtphosphaten (layered phosphates - mit 2-D-Strukturen) oder 3-D-Phosphaten (Zeolith-Typ) bestehen.

In einer besonders bevorzugten Ausführungsform der vorliegenden Erfindung ist in den Flammschutzmittelzusammensetzungen enthaltend die Komponente (a), (A-H)⁽⁺⁾ = (II-1) und M=Zn oder Al.

In einem nicht beanspruchtem Beispiel kann die Zusammensetzung weiterhin wenigstens eine sich von Triazin-interkaliertem (Metall)Phosphat unterscheidende Flammschutzmittelkomponente (b) umfassen, wobei die Komponente (b) mindestens eine Metallverbindung, die kein Metallphosphat der Komponente (a) ist, oder/und mindestens eine metallfreie Phosphorverbindung.

Diese mindestens eine Metallverbindung (b) ist vorzugsweise ein Metalloxid, ein Metallhydroxid, ein Metallphosphat, ein Metallpyrophosphat, ein Hydrotalcit, ein kationisch oder anionisch modifizierter Organoclay, ein Stannat oder Molybdat-Salz, ein Metallborat oder Metall-Phosphinat der Formel (III): 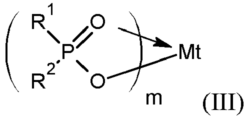 wobei R¹ und R² Wasserstoff oder ein geradkettiger oder ein verzweigter C₁ - C₆Alkylrest oder ein Phenylrest; und Mt = Ca, Mg, Zn oder Al und m = 2 oder 3 bzw. ein Hypophosphitsalz der Formel M^{m+}[H₂PO₂]ₘ^{m-} (M = Al, Ca, Mg und Zn sowie m = 2 und 3) ist.

Unter Organoclays versteht man organophilmodifizierte Tonmineralien (haupsächlich Montmorillonite ) auf Basis von Kationenaustausch wie Triethanol-Talg-Ammonium-Montmorillonit und Triethanol-Talg-Ammonium-Hektorit (Dr. G. Beyer; Konf. Fire Resistance in Plastics 2007*).* Anionic Organoclays bedeuten organophil-modifizierte Hydrotalcite auf Basis von Anionenaustausch mit Alkalirosinaten, ungesättigten und gesättigten Fettsäuresalzen sowie langkettig alkylsubstituierten Sulfonaten und Sulfaten.

Besonders bevorzugt als Metalloxid ist Diantimontrioxid, Diantimontetroxid, Diantimonpentoxid oder Zinkoxid.

Besonders bevorzugt als Metallhydroxid sind Aluminiumhydroxid (ATH) bzw. Gibbsit (Hydrargillit), Aluminiumoxohydroxid (Boehmit) und Magnesiumhydroxid (MDH, Brucit) sowie Hydromagnesit. Neben Gibbsit und Boehmit sind auch die anderen Modifikationen von Aluminiumhydroxiden, nämlich Bayerit, Nordstrandit und Diaspor anzuführen.

Als Metallphosphat sind Metallpyrophosphate bevorzugt. Besonders bevorzugt sind Aluminium- und Zinkpyrophosphat sowie Zink- und Aluminiumtriphosphat ebenso wie Aluminium-metaphosphat und Aluminium-orthophosphat.

Als Hydrotalcit bevorzugt sind Magnesium-Aluminium- und Calcium-Aluminium-Hydroxocarbonat.

Unter den kationisch- oder anionisch-modifizierten Organoclays sind die alkylsulfat- oder fettsäurecarboxylat- modifizierten Hydrotalcite oder langkettig quaternär-ammoniummodifizierten Tonmineralien besonders bevorzugt.

Unter den Stannat- und Molybdatsalzen sind Zinkstannat, Zinkhydroxystannat, Ammoniumheptamolybdat und Ammoniumocatmolybdat besonders bevorzugt. Ebenso zu benennen sind andere Molybdate (auch Polymolybdate) wie Calciumzinkmolybdat, basisches Zinkmolybdat und Calciummolybdat.

Als Borate bevorzugt sind Alkali- und Erdalkaliborate sowie Zinkborat. Weiterhin anzu führen sind Aluminiumborat, Bariumborat, Calciumborat, Magnesiumborat, Manganborat, Melaminborat, Kaliumborat sowie Zinkborphosphat.

Unter den Metallphosphinaten sind bevorzugt Ca-, Mg-, Zn- oder Al-Phosphinate. Besonders bevorzugt sind Ca-, Mg-, Zn- oder Al-Phenyl(benzol)phosphinat und Ca-, Mg-, Zn- oder Al- diethyl(ethan)phosphinat.

Unter den Hypophosphiten sind besonders bevorzugt, das Mg-, Ca-, Zn und Al-Salz.

Eine weitere Bevorzugung der Erfindung betrifft Flammschutzmittelzusammensetzungen enthaltend als Komponente (b) mindestens eine metallfreie Phosphorverbindung.

Diese mindestens eine metallfreie Phosphorverbindung (b) ist roter Phosphor, ein oligomerer Phosphatester, ein oligomerer Phosphonatester, ein zyklischer Phosphonatester, ein Thiopyrophosphorsäureester, Melaminpyrophosphat, Melaminpolyphosphat, Ammoniumpolyphosphat, Melaminium-Phenylphosphonat sowie dessen Halbestersalz (WO2010/063623), Melamin-Benzolphosphinat (WO2010/057851), Hydroxyalkyl-Phosphinoxide (WO2009/034023), Tetrakis-hydroxymethyl-Phosphoniumsalze und Phospholan- bzw. Phosphol-Derivate sowie Bisphosphoramidate mit Piperazin als Brückenglied oder ein Phosphonitester.

Oligomere Phosphatester besitzen Formel (IV) oder Formel (V): 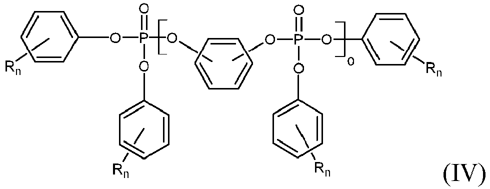 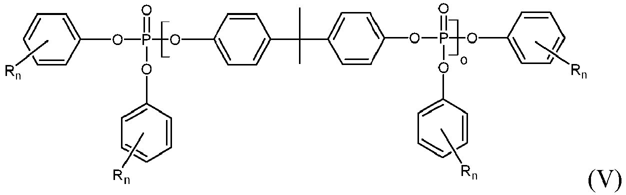 wobei jedes R jeweils unabhängig Wasserstoff, C₁ - C₄ -Alkyl oder Hydroxy, n = 1 bis 3 und o 1 bis 10 ist.

Besonders bevorzugt sind das Oligomer mit Rₙ = H und Resorcin bzw. Hydrochinon als Bestandteil des Brückengliedes sowie Rₙ = H und Bisphenol-A oder Bisphenol-F als Bestandteil des Brückengliedes.

Oligomere Phosphonatester sind vorzugsweise durch Formel (VI) charakterisiert: 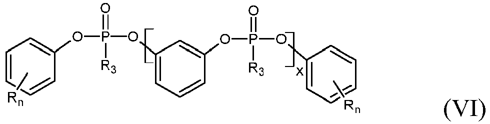 wobei R³ = Methyl oder Phenyl und x 1 bis 20 ist und R, n die oben angegebene Bedeutung besitzen.

Besonders bevorzugt sind das Oligomer mit Rₙ = H und Resorcin bzw. Hydrochinon als Bestandteil des Brückengliedes.

Zyklische Phosphonatester weisen vorzugsweise folgende Formel (VII) auf: 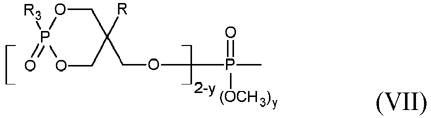 wobei y = 0 oder 2 ist. Besonders bevorzugt ist Bis[5-ethyl-2-methyl-1,3,2-dioxaphosphorinan-5-yl)methyl]methyl phosphonate P,P'-dioxid.

Thiopyrophosphorsäureester sind vorzugsweise durch folgende Formel (VIII) charakterisiert: 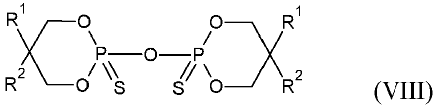 Besonders bevorzugt ist 2,2'-Oxybis[5,5-dimethyl-1,3,2-dioxaphosphorinan]2,2'-disulfid.

Unter den Hydroxyalkyl-Phosphinoxiden sind bevorzugt Isobutyl-bis-hydroxymethyl-Phosphinoxid sowie dessen Kombination mit Epoxyharzen (WO-A 2009/034023).

Unter den Tetrakis-hydroxyalkyl-Phosphoniumsalzen sind die Tetrakis-hydroxymethyl-Phosphoniumsalze besonders bevorzugt.

Unter den Phospholan- bzw. Phosphol-Derivaten sind Dihydrophosphol(oxid)-Derivate und Phospholan (oxid)-derivate sowie deren Salze (EP 089 296 und EP 1024 166) besonders bevorzugt.

Besonders bevorzugt unter den Bisphosphoramidaten sind die Bis-di-ortho-xylylester mit Piperazin als Brückenglied.

Unter den Phosphonitestern sind bevorzugt, Benzol-Phosphinsäurephenylester und dessen PH-fuktionalisierte Derivate und DOPO-Derivate.

Als DOPO-Derivate (9,10-Dihydro-9-oxa-10-Phosphaphenanthren-10-oxid-Derivate oder 6H-Dibenzo(c,e) (1,2)-oxaphosphorin-6-oxid)-Derivate (wobei PH-funktionalisierte Derivate bevorzugt sind) sind folgende Verbindungen strukturell dargestellt (vgl. WO-A 2008/119693):

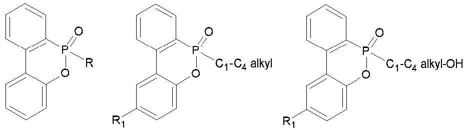

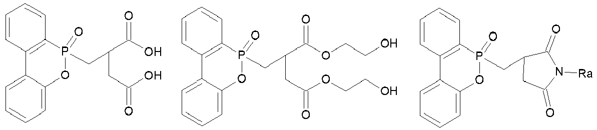

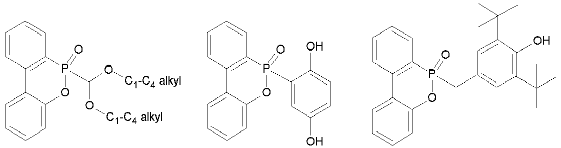

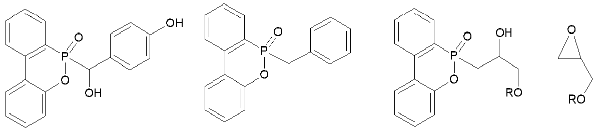

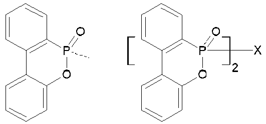

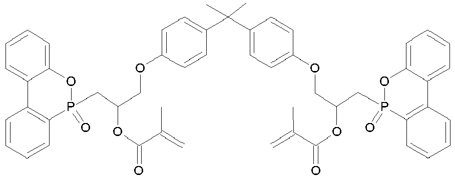

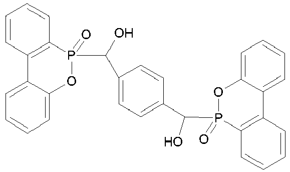

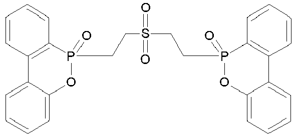

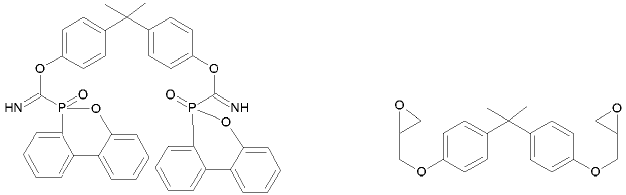

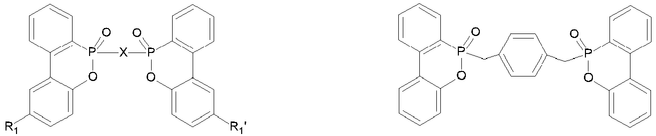

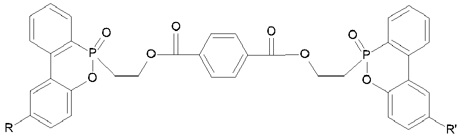

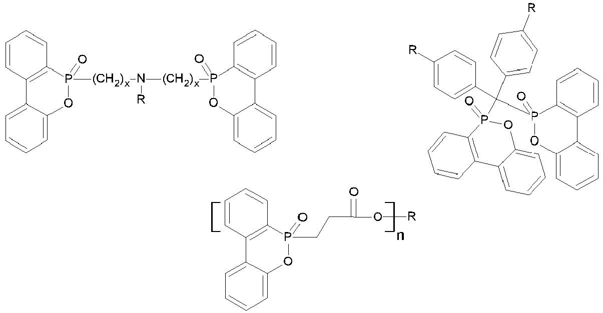

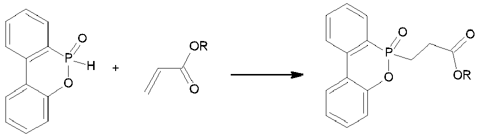

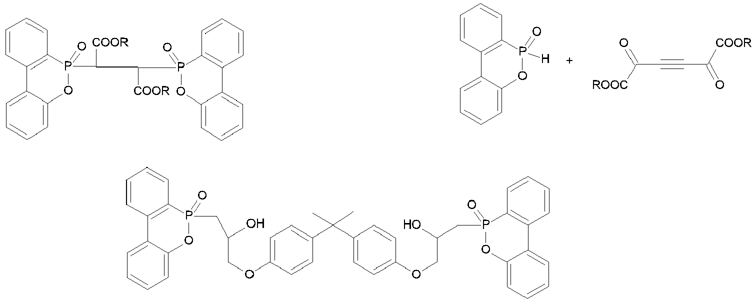

Besonders bevorzugt sind:

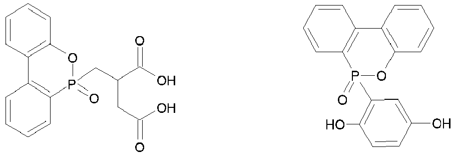

Anstelle von DOPO kann auch Dihydro-oxa-phospha-anthracenoxid(on) treten. Eine Übersicht hierzu ist aus WO-A 2008/119693 zu entnehmen.

Als weitere Additive (Synergisten) sind zu nennen: Polyole, Aminouracile, Trishydroxyethylisocyanurat (THEIC), Melamin(iso)cyanurat, POSS-Verbindungen und Blähgraphit.

Von den Polyolen sind Pentaerythrit, Dipentaerythrit und Tripentaerythrit besonders bevorzugt.

Von den Aminouracilen sind 1-Methyl-6-aminouracil und 1,3-Dimethyl-6-aminouracil besonders bevorzugt.

POSS-Verbindungen (**P**olyhedral **o**ligomeric **S**ilsesquioxanes) und Derivate werden näher beschrieben in POLYMER, Vol. 46, pp 7855-7866. Bevorzugt sind hierbei POSS-Derivate auf Methylsiloxan-Basis.

Ferner könne auch Tris-hydroxyethyl-isocyanurat-polyterephthalate sowie Triazin-Polymere mit Piperazin- 1,4-diyl-Brückengliedern und Morpholin-1-yl-Endgruppen zugegen sein.

Weiterhin können folgende Zusatzstoffen zugegen sein: Bis-Azinpentaerythritdiphosphat-Salze, Hexa-aryloxy-triphosphazene, Poly-aryloxy-phosphazene und Siloxane (R₂SiO)ᵣ oder (RSiO_{1,5})ᵣ.

Metalloxide wie Titandioxid, Siliziumdioxid; Tonmineralien wie Kaolinit, Muskovit, Pyrophyllit, Bentonit und Talk oder andere Mineralien wie Wollastonit, Quarz, Glimmer, Feldspat.

Ferner können im Polymeren zusätzlich Dolomit, Bentonit, Huntit, oder Kieselsäuren und deren natürliche bzw. synthetische Silikatminerale enthalten sein.

Außerdem können einem Polymer zusätzlich zu dem mindestens einen erfindungsgemäßen Metall-Phosphat Schaumbildner zugesetzt werden. Als Schaumbildner seien genannt: Melamin, Melaminformaldehydharze, Harnstoffderivate wie Harnstoff, Thioharnstoff, Guanamine, Benzoguanamin, Azetoguanamin und Succinylguanamin, Dizyandiamid, Guanidin und Guanidinsulfamat sowie andere Guanidinsalze bzw. Allantoine und Glykolurile.

Darüber hinaus kann ein Polymer, enthaltend das mindestens eine erfindungsgemäße Metall-Phosphat, auch Antidripping-Mittel, insbesondere auf Polytetrafluoroethylen-Basis enthalten. Die Konzentration solcher Antidrippingmittel beträgt 0,01 bis 15 Gew.-% bezogen auf das zu verarbeitende Polymer.

Zusätzlich können Polymeren, enthaltend das mindestens eine erfindungsgemäße Metall-Phosphat, auch weitere Komponenten zugegeben werden, wie z.B. Füllstoffe und Verstärkungsmittel, wie Glasfasern, Glasperlen oder mineralische Zusätze wie Kreide. Als weitere Additive können Antioxidantien, Lichtstabilisatoren, Gleitmittel, Pigmente, Nukleierungsagentien und Antistatika fungieren.

Die vorliegende Erfindung betrifft außerdem die Verwendung der erfindungsgemäßen Triazin-interkalierten Metall-Phosphate mit offenen Gerüststrukturen als Flammschutzmittel in einem Polymer, Papier, Textilien oder Wood Plastic Composites (WPC).

Die erfindungsgemäßen Flammschutzmittel sind bestens geeignet, synthetischen, insbesondere thermoplastischen Polymeren, Flammschutzeigenschaften zu verleihen.

Eine besondere Ausführungsform der Erfindung betrifft die Verwendung des mindestens einen erfindungsgemäßen Metall-Phosphats in einem Polymer als Flammschutzmittel, wobei das Polymer ein Thermoplast der vorzugsweise ausgewählt ist aus der Gruppe bestehend aus Polyamid, Polycarbonat, Polyolefin, Polystyrol, Polyester, Polyvinylchlorid, Polyvinylalkohol, ABS und Polyurethan oder ein Duroplast ist, der vorzugsweise ausgewählt ist aus der Gruppe bestehend aus Epoxidharz (mit Härter), Phenolharz und Melaminharz.

Ist das Polymer, in dem das mindestens eine erfindungsgemäße Metall-Phosphat als Flammschutzmittel eingesetzt wird, ein Thermoplast, so ist Polyamid, Polyurethan, Polystyrol, Polyolefin oder Polyester bevorzugt.

Ist das Polymer, in dem das mindestens eine erfindungsgemäße Metall-Phosphat als Flammschutzmittel eingesetzt wird, ein Duroplast, so ist Epoxidharz bevorzugt.

Es können auch Mischungen von einem oder mehreren Polymeren, insbesondere Thermo- und/oder Duroplasten, in denen das erfindungsgemäße Metall-Phosphat als Flammschutzmittel eingesetzt wird, verwendet werden.

Beispiele für solche Polymere sind:
1) Polymere von Mono- und Diolefinen, z.B. Polypropylen, Polyisobutylen, Polybuten-1, Poly-4-methylpenten-1, Polyvinylcyclohexan, Polyisopren oder Polybutadien und Polymerisate von Cycloolefinen, z.B. von Cyklopenten oder Norbornen und Polyethylen (auch vernetzt), z.B. High Density Polyethylen (HDPE) oder High Molecular Weight (HDPE-HMW), High Density Polyethylen mit Ultra-High Molecular Weight (HDPE-UHMW), Medium Density Polyethylen (MDPE), Low Density Polyethylen (LDPE) und Linear Low Density Polyethylen (LLDPE), (VLDPE) und (ULDPE) sowie Copolymere von Ethylen und Vinylacetat (EVA);
2) Polystyrole, Poly(p-methylstyrol), Poly(a-methylstyrol);
3) Copolymere sowie Propfcopolymere von Polybutadien-Styrol oder Polybutadien und (Meth)Acrylnitril wie z.B. ABS und MBS;
4) Halogenhaltige Polymere z.B. Polychloropren, Polyvinylchlorid (PVC); Polyvinylidenchlorid (PVDC), Copolymere von Vinylchlorid/Vinylidenchlorid, Vinylchlorid/Vinylacetat oder Vinylchlorid/Vinylacetat;
5) Poly(meth)acrylate, Polymethylmethacrylate (PMMA), Polyacrylamid und Polyacrylnitril (PAN);
6) Polymere von ungesättigten Alkoholen und Aminen oder ihren Azylderivaten bzw. Azetalen, wie z.B. Polyvinylalkohol (PVA), Polyvinylacetate, -stearate, -benzoate oder maleate, Polyvinylbutyral, Polyallylphthalate und Polyallylmelamine;
7) Homo- und Copolymere von cyclischen Ethern, wie Polyalkylenglykole, Polyethylenoxide, Polypropylenoxide und deren Copolymere mit Bisglycidylethern;
8) Polyazetale, wie Polyoxymethylene (POM) sowie Polyurethan und Acrylat modifizierte Polyazetale;
9) Polyphenylenoxide und -sulfide und deren Gemische mit Styrolpolymeren oder Polyamiden;
10) Polyamide und Copolyamide hergeleitet von Diaminen und Dicarbonsäuren und/oder von Aminocarbonsäuren oder den entsprechenden Laktamen, wie z.B. Polyamid 4, Polyamid 6, Polyamid 6/6, 6/10, 6/9, 6/12, 12/12, Polyamid 11, Polyamid 12, aromatische Polyamide hergeleitet vom m-Xylylendiamin und Adipinsäure und Copolyamide modifiziert mit EPDM oder ABS. Beispiele von Polyamiden und Copolyamiden sind hergeleitet von ε-Kaprolaktam, Adipinsäure, Sebacinsäure, Dodekansäure, Isophthalsäure, Terephthalsäure, Hexamethylendiamin, Tetramethylendiamin, 2-Methyl-pentamethylendiamin, 2,2,4-Trimethylhexamethylendiamin, 2,4,4-Trimethylhexamethylendiamin, m-Xylylendiamin oder Bis(3-Methyl-4-aminozyklohexyl)methan;
11) Polyharnstoffe, Polyimide, Polyamidimide, Polyetherimide, Polyesterimide, Polyhydantoine und Polybenzimidazole;
12) Polyester hergeleitet von Dicarbonsäuren und Dialkoholen und/oder Hydroxycarbonsäuren oder den entsprechenden Laktonen, wie z.B. Polyethylenterephthalat, Polypropylenterephthalat, Polybutylenterephthalat, Poly-1,4-dimethylzyklohexanterephthalat, Polyalkylennaphthalat (PAN) und Polyhydroxybenzoate, Polymilchsäureester und Polyglykolsäureester;
13) Polycarbonate und Polyestercarbonate;
14) Polyketone;
15) Mischungen bzw. Legierungen von o.g. Polymeren z.B. PP/EPDM, PA/EPDM oder ABS, PVC/EVA, PVC/ABS, PBC/MBS, PC/ABS, PBTP/ABS, PC/AS, PC/PBT, PVC/CPE, PVC/Acrylat, POM/thermoplastisches PUR, PC/thermoplastisches PUR, POM/Acrylat, POM/MBS, PPO/HIPS, PPO/PA6.6 und Copolymere, PA/HDPE, PA/PP, PA/PPO, PBT/PC/ABS oder PBT/PET/PC, sowie TPE-O, TPE-S und TPE-E;
16) Duroplaste wie PF, MF oder UF oder Mischungen davon;
17) Epoxidharze - Thermoplaste und Duroplaste;
18) Phenolharze;
19) Wood-Plastic-Composites (WPC) sowie Polymere auf PLA-, PHB- und Stärke-Basis.

Die Konzentration des mindestens einen beanspruchten Triazin-interkalierten Metall-Phosphates (a) und der nicht beanspruchten Komponente (b) in einem Polymer oder einer Polymermischung betragen vorzugsweise 0,1 bis 60 Gew.-% bezogen auf das zu verarbeitende Polymer.

Das durch Zusatz des mindestens einen erfindungsgemäßen Metall-Phosphats so flammgeschütze Material kann zu Fasern, Folien, Gussartikeln verarbeitet sowie zur Behandlung von Oberflächen verwendet werden.

Das mindestens eine erfindungsgemäße Metall-Phosphat kann auch zur Oberflächenbehandlung (Imprägnierung) von Fasern, Folien, Textilien oder andere technische Materialien verwendet werden.

Dementsprechend betrifft die vorliegende Erfindung die Verwendung einer erfindungsgemäßen Zusammensetzung in einem als Flammschutzmittel in einem Polymer, Papier, Textilien oder Wood Plastic Composites (WPC). Insbesondere handelt es sich bei dem Polymer um einen Thermoplasten, vorzugsweise ausgewählt aus der Gruppe bestehend aus Polyamid, Polycarbonat, Polyolefin, Polystyrol, Polyester, Polyvinylchlorid, Polyvinylalkohol, ABS und Polyurethan oder ein Duroplast ist, vorzugsweise ausgewählt aus der Gruppe bestehend aus Epoxidharz, Phenolharz und Melaminharz.

Beschrieben, allerdings nicht beansprucht wird die Verwendung der erfindungsgemäßen Triazin-interkalierten Metall -Phosphate mit offenen Gerüststrukturen für die Herstellung von Lacken, Klebstoffen, Gießharzen, Beschichtungen, Thixotropiemittel und Flammschutzmittel für Polymere.

Weiterhin beschrieben, jedoch nicht beansprucht wird die Verwendung des mindestens einen erfindungsgemäßen Metall-Phosphats als Füllstoff in Polymeren.

Ein beispielhaftes Verfahren zur Herstellung des erfindungsgemäßen Metall-Phosphats, wobei eine Substanz (A) mit einem aziden Metall-Phosphat der Formel Hₐ⁽⁺⁾[M_{b}^{m+} (H₂PO₄)ₓ₁⁽⁻⁾(HPO₄)ₓ₂²⁽⁻⁾(PO₄)ₓ₃³⁽⁻⁾(PO₃)_{y}⁽⁻⁾]^{(a-)}∗pH₂O umgesetzt wird.

Insbesondere umfasst das Verfahren zur Herstellung der oben genannten Verbindungen den Schritt des Umsetzens einer Verbindung (A), wobei (A) ein Triazin der Formeln (II-4), (II-5) oder (II-6) 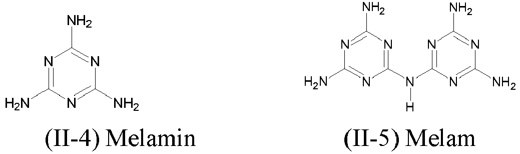 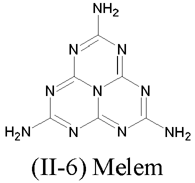 ist mit einem aziden Metall-Phosphat der Formel Hₐ⁽⁺⁾[M_{b}^{m+}(H₂PO₄)ₓ₁⁽⁻⁾(HPO₄)ₓ₂²⁽⁻⁾ (PO₄)ₓ₃³⁽⁻⁾(PO₃)_{y}⁽⁽⁻⁾]^{(a-)∗}pH₂O, wobei jedes M = Al.

In einem erfindungsgemäßen Verfahren zur Herstellung des erfindungsgemäßen Metall-Phosphats kann die Umsetzung in Wasser und vorzugsweise zwischen 20 und 90 °C, besonders bevorzugt zwischen 20 und 60 °C und ganz besonders bevorzugt zwischen 20 und 40 °C stattfinden.

Insbesondere zeichnen sich solche Verbindungen dadurch aus, dass die Brutto-Zusammensetzung ein Melamin-Aluminiumphosphat [(Melamin-H)₂⁺ [AlP₃O₁₀]²⁽⁻⁾]_{z} ist, folgende ³¹P-MAS-NMR-Verschiebungen (δ-Werte) aufweist: -10,6ppm, -22,0ppm,-24,5ppm und -27,6ppm und im ²⁷Al-NMR-Spektrum eine einzige Verschiebung um 40ppm zeigt. Insbesondere ist die Brutto-Zusammensetzung ein Melamin-Zinkphosphat [(Melamin-H)₂⁺ [ZnP₂O_{7]}²⁽⁻⁾]_{z} mit folgenden ³¹P-MAS-NMR-Verschiebungen (δ-Werte): +6,2ppm, +3,7ppm, +2,0ppm, -2,5ppm, -5,5ppm, -8,2ppm, -10,7ppm, -12,1ppm, -22,2ppm und -24,7ppm.

Das jeweilige Metall-Phosphat kann z.B. durch Vormischung in Form von Pulver und/oder Granulat in einem Mixer und dann durch Homogenisieren in einer Polymerschmelze durch Compoundieren (u.a. in einem Doppelschneckenextruder) hergestellt werden. Das Metall-Phosphat kann evtl. auch bei der Verarbeitung direkt zugegeben werden.

Als Metall -Phosphate für die Herstellung von Triazin-interkalierten Metall-Phosphaten mit offenen Gerüststrukturen kommen insbesondere Schicht-Phosphate der Formeln M(H₂PO₄)₃ und M(H₂PO₄)₂ (M = Al, oder Zn) und kondensierte Phosphate wie Triphosphate der Formeln H₂AlP₃O₁₀ in Frage.

Am besten werden die Systeme jedoch über eine Reaktion mit Melamin als Templat in wässriger acider Metallsalzlösung hergestellt. Ein Alternativ-Verfahren besteht in der Umsetzung von Triazinphosphaten mit wässrigen Metallsalz-Lösungen (in Anlehnung an Angew. Chem., 1999, 111, 3688-3692).

Die so hergestellten Metall-Phosphate mit offenen Gerüststrukturen weisen Ortho-Phosphat (HₓPO₄-Typ mit x = 2, 1 oder 0), Pyrophosphat oder Triphosphat als Komplexliganden auf, wobei zwischen die Gitterschichten oder in die Hohlräume Melamin in protonierter Form (Melamin-Kation) interkaliert wird und bei Schichtstrukturen eine Aufweitung der Schichtabstände stattfindet.

In der weiteren Verarbeitung werden die erfindungsgemäßen Triazin-interkalierten Metall-Phosphate in eine geeignete Polymermatrix eingearbeitet. Geeignete Polymere, welche als Substrat verwendet werden können, sind an sich bekannt. Für die Einarbeitung bevorzugt sind thermoplastische Polymere und duroplastische Polymersysteme, Kautschuke und Textilien.

Melamin ist als Interkalat bevorzugt.

Mit Ortho-Phosphat als Liganden lassen sich die neuen Interkalate beispielhaft wie folgt darstellen, wobei **(A** - **H)**⁽⁺⁾ (Mel-H)⁽⁺⁾ (Melamin-Kation) ist:
1. (Mel-H)₂⁽⁺⁾[Zn²⁽⁺⁾(H₂PO₄)₂⁽⁻⁾(HPO₄)²⁽⁻⁾]²⁽⁻⁾
2. (Mel-H)⁽⁺⁾[Zn₂²⁽⁺⁾(H₂PO₄)₂⁽⁻⁾(PO₄)³⁽⁻⁾]⁽⁻⁾
3. (Mel-H)₂⁽⁺⁾[Zn₂²⁽⁺⁾(H₂PO₄)₂⁽⁻⁾(HPO₄)₂²⁽⁻⁾]²⁽⁻⁾
4. (Mel-H) ⁽⁺⁾[Zn²⁽⁺⁾(H₂PO₄)⁽⁻⁾(HPO₄)²⁽⁻⁾]⁽⁻⁾
5. (Mel-H)₄⁽⁺⁾[Zn₂²⁽⁺⁾(PO₄)³⁽⁻⁾(PO₃)₅⁽⁻⁾]⁴⁽⁻⁾ (Mischtyp aus Meta- und Pyrophosphat)
6. (Mel-H)⁽⁺⁾[Zn₂²⁽⁺⁾(PO₄)³⁽⁻⁾(PO₃)₂⁽⁻⁾]⁽⁻⁾ (Mischtyp aus Meta- und Pyrophosphat)
7. (Mel-H)₂⁽⁺⁾[Zn₂²⁽⁺⁾(PO₄)³⁽⁻⁾(PO₃)₃⁽⁻⁾]²⁽⁻⁾ (Mischtyp aus Meta- und Pyrophosphat)
8. (Mel-H)₄⁽⁺⁾[Zn₁₂²⁽⁺⁾(PO₄)₉³⁽⁻⁾(PO₃)⁽⁻⁾]⁴⁽⁻⁾ (Mischtyp aus Ortho- und Pyrophosphat)
9. (Mel-H)₄⁽⁺⁾[Zn₆²⁽⁺⁾(PO₄)₅³⁽⁻⁾(PO₃)⁽⁻⁾]⁴⁽⁻⁾ (Mischtyp aus Ortho- und Pyrophsophat)
10. (Mel-H)₂⁽⁺⁾[Zn₄²⁽⁺⁾(PO₄)₃³⁽⁻⁾(PO₃)⁽⁻⁾]²⁽⁻⁾ (Mischtyp aus Ortho- und Pyrophosphat)
11. (Mel-H)₂⁽⁺⁾[Zn₄²⁽⁺⁾(PO₄)₃³⁽⁻⁾(PO₃)⁽⁻⁾]²⁽⁻⁾ (Mischtyp aus Ortho- und Pyrophosphat)
12. (Mel-H)₂⁽⁺⁾[Zn₂²⁽⁺⁾(PO₄)³⁽⁻⁾(PO₃)₃⁽⁻⁾]²⁽⁻⁾ (Mischtyp aus Meta- und Pyrophosphat)
13. (Mel-H)₄⁽⁺⁾[Al₁₀³⁽⁺⁾(PO₄)₁₁³⁽⁻⁾(PO₃)⁽⁻⁾]⁴⁽⁻⁾ (Mischtyp aus Ortho- und Pyrophosphat)
14. (Mel-H)₄⁽⁺⁾[Al₈³⁽⁺⁾(PO₄)₉³⁽⁻⁾(PO₃)⁽⁻⁾]⁴⁽⁻⁾ (Mischtyp aus Ortho- und Pyrophosphat)
15. (Mel-H)₂⁽⁺⁾[Al³⁽⁺⁾(H₂PO₄)⁽⁻⁾(HPO₄)₂²⁽⁻⁾]²⁽⁻⁾
16. (Mel-H)₂⁽⁺⁾[Al³⁽⁺⁾(PO₄)³⁽⁻⁾(PO₃)₂⁽⁻⁾]²⁽⁻⁾ (Triphosphat-Typ)
wobei Aquo(Komplex)-Wasser durch thermische Behandlung entfernt werden kann.

Besonders bevorzugt sind 15 und 16. Ganz besonders bevorzugt ist 16.

Weiterhin wird ein Verfahren zur Herstellung eines flammgeschützten, verformbaren Polymers beschrieben, wobei das mindestens eine erfindungsgemäße, Triazin-interkalierte Metall-Phosphat in dem Polymer exfoliert wird.

Ein weiterer Gegenstand der Erfindung ist die Erzielung von antikorrosiven Schutzeffektes durch Coating von Metalloberflächen.

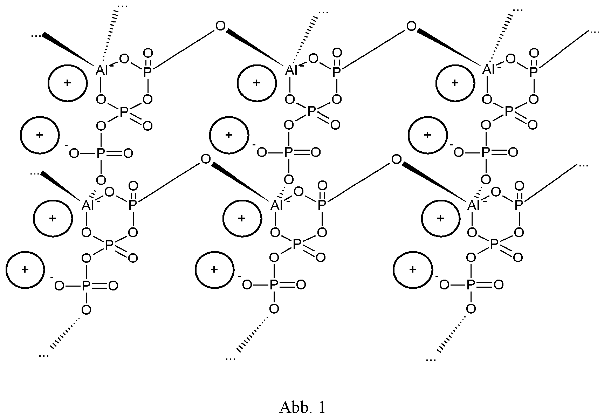

Abb. 1 zeigt beispielhaft einen Gitterausschnitt aus einem Interkalationsmodel von Melamin in Aluminiumtriphosphat (AlH₂P₃O₁₀)-Schichten (⊕ = Melaminium-Kation).

Die Erfindung wird durch nachfolgende Beispiele näher erläutert.

Eingesetzte Substanzen: Melamin (DSM); Aluminium-tris-dihydrogenphosphat (50%ige Lösung in Wasser) (PRAYON Deutschland), Zinkoxid, ortho-Phosphorsäure (ALDRICH)

### Beispiel 1: Synthese von Bis-Melamin-alumo-dihydrogenphosphat-bis hydrogenphosphat

### (Produkt A) -Precursor-Verbindung

(C₃H₇N₆)₂⁽⁺⁾[Al(H₂PO₄)(HPO₄)₂]²⁽⁻⁾

(**a** = 2, **M** = Al, **b** = 1, **m** = 3, **x₁** = 1, **x₂** = 2, **x₃** = 0, **y** = 0, **p** = 0)

100,9 g (0,8 Mol) Melamin werden in 2,4 1 Wasser unter Rühren in der Wärme (40 bis 60 °C) gelöst. In dieser Lösung tropft man 254,4 g (0,4 Mol) Aluminium-tris-dihydrogenphosphat (50%ige Lösung in Wasser), wobei sich ein dicker Brei bildet. Anschließend wird 30 Min. nachgerührt, auf Raumtemperatur abgekühlt, der entstandene weiße Niederschlag abgesaugt, mit Wasser nachgewaschen und bei 120 °C gewichtskonstant getrocknet.

Ausbeute: 211,7 g entspricht 92,8 % d.Th.

Elementar-Analyse: C: **12,7** % (12,6 %); H: **3,3**% (3,2 %); N: **29,9**% (29,5 %); Al: **4,7**% (4,7 %); P: **16,4** % (16,3 %) (Theorie-Werte)

### Beispiel 2: Synthese von Bis-Melamin-alumo-triphosphat

### (Produkt B)

(C₃H₇N₆)₂⁽⁺⁾[Al³⁽⁺⁾(PO₄)³⁽⁻⁾(PO₃)₂⁽⁻⁾]²⁽⁻⁾

(**a** = 2, **M** = Al, **b** = 1, **m** = 3, **x₁** = 0, **x₂** = 0, **x₃** = 1, **y** = 2, **p** = 0)

**Produkt (A)** wird unter öfterem Mischen 5 h bei 280 °C nahezu gewichtskonstant getempert. Das resultierende weiße Produkt besitzt folgende Zusammensetzung:

Elementar-Analyse: C: **13,5** % (13,5 %); H: **2,6** % (2,6 %); N: **30,1** % (31,5); Al: **5,1** % (5,1%); P: **17,5** % (17,4 %) (Theorie-Werte)

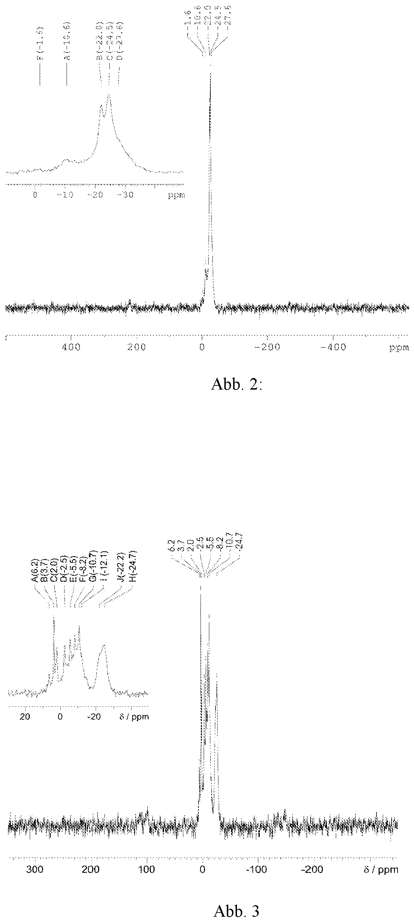

³¹P-MAS-NMR-Verschiebungen (δ-Werte): -10,6ppm, -22,0ppm, -24,5ppm und -27,6ppm (siehe Abb. 2). Hierbei zeigt Abb. 2 das quantitatives ³¹P-NMR Spektrum von Bis-Melamin-alumo-triphosphat (Produkt B) (v_{MAS}=20KHz, ¹H-entkoppelt)).

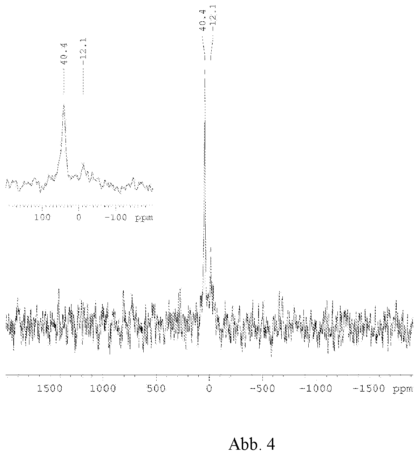

²⁷Al-NMR-Spektrum: einzige Verschiebung um 40ppm (s. Abb. 4, v_{MAS}=20KHz).

### Vergleichsbeispiel 3: Synthese von Tris-Melamin-alumo-tris-hydrogenphosphatdihydrat

### (Produkt C)-Precursor-Verbindung

(C₃H₇N₆)₃⁽⁺⁾[Al(HPO₄)₃]³⁽⁻⁾∗2H₂O

(**a** = 3, **M** = Al, **b** = 1, **m**= 3, **x₁** = 0, **x₂** = 3, **x₃** = 0, **y** = 0, **p** =2).

94,6 g (0,75 Mol) Melamin werden in 2,3 1 Wasser unter Rühren in der Hitze gelöst. In diese Lösung tropft man 159,0 g (0,25 Mol) Aluminium-tris-dihydrogenphosphat (50%ige Lösung in Wasser), wobei sich ein voluminöser Brei bildet. Anschließend wird 30 Minuten nachgerührt, auf Raumtemperatur abgekühlt, der entstandene weiße Niederschlag abgesaugt, zweimal mit Wasser nachgewaschen und bei 120 °C gewichtskonstant getrocknet. Ausbeute: 174,0 g entspr. 95,0 % d.Th.

Elementar-Analyse: C: **14,8** % (14,8 %); H: **3,5** % (3,9 %); N: 33,**8** % (34,4 %) (Theorie-Werte)

### Beispiel 4: Synthese von Produkt B ausgehend von Produkt C.

Herstellung von **Produkt C** wie in Beispiel 3, aber mit nachfolgender Temperung 5 h bei 210 °C. Es resultiert als Vorstufe Tris-melamin-alumo-tris-dihydrogenphosphatmonohydrat.

(C₃H₇N₆)₃⁽⁺⁾(HPO₄)₃]³⁽⁻⁾∗H₂O.

Ausbeute: 165,7 g entspr. 92,8 % d. Th.

Elementar-Analyse: C: **15,1** % (15,1 %); H: **4,3** % (3,7 %); N: **35,1** % (35,3 %); (Theorie-Werte)

Aus dieser Vorstufe wird durch erneutes Tempern bei 280 °C, 6 h **Produkt B** erhalten, wobei eine Gewichtsabnahme von 25 % stattfindet. Es resultiert Bis-melamin-alumo-triphosphat

(C₃H₇N₆)₂⁽⁺⁾[Al³⁽⁺⁾(PO₄)³⁽⁻⁾(PO₃)₂⁽⁻⁾]²⁽⁻⁾ (Ausbeute quant.)

Elementar-Analyse: **C: 13,4** % (13,5 %); **H: 4,0** % (2,6 %); **N: 29,7** % (31,5 %); (Theorie-Werte)

Hieraus ist ersichtlich, dass zu **Produkt B** durch Verwendung von **Produkt C** auch ein Alternativ-Zugang möglich ist. Dieses Verfahren ist jedoch in der Praxis unwirtschaftlich, da ca. ein Drittel des eingesetzten Melamins durch Temperung wieder entfernt werden muss.

Verzichtet man jedoch auf die Temperung, so ist eine Einarbeitung in Polyamide, Polycarbonate und Polyester stark erschwert, da nennenswerte Mengen an Melamin absublimieren. Bei Einsatz des nach Bespiel 2 hergestellten Produktes B treten diese Schwierigkeiten jedoch nicht auf.

### Vergleichsbeispiel 5: Synthese von Bis-Melamin-zinko-diphosphat

### (Produkt D)

(C₃H₇N₆)₂⁽⁺⁾[Zn²⁽⁺⁾(PO₄)³⁽⁻⁾(PO₃)⁽⁻⁾]²⁽⁻⁾

(**a** = 2, **M** = Zn, **b** = 1, **m**= 2, **x₁** = 0, **x₂** = 0, **x₃** = 1, **y** = 1, **p** =0).

Produkt D erhalten nach obiger Vorschrift, wird 5 h bei 280 °C getrocknet, wobei eine Gewichtsabnahme von ca. 6,0 % stattfindet.

Elementar-Analyse: C: **15,1** % (14,6 %); H: **2,8** % (2,9 %); N: **34,0** % (34,1 %); Zn: 12,6 % (13,3 %); P: 12,2 % (12,2 %). (Theorie-Werte)

³¹P-MAS-NMR-Verschiebungen (δ-Werte): +6,2ppm, +3,7ppm, +2,0ppm, -2,5ppm,-5,5ppm, -8,2ppm, -10,7ppm, -12,1ppm, -22,2ppm und -24,7ppm. (s. Abb. 3). Hierbei zeigt Abb. 3 das quantitative ³¹P-NMR Spektrum von Bis-Melamin-zinko-diphosphat (Produkt D) (v_{MAS}=20KHz).

### Beispiel 6: Statische thermische Behandlung der Precursor-Produkten A und C:

Die Ergebnisse sind in der Tab. 1. zusammengefasst.

**Tabelle 1: Thermische Behandlung von Precursor-Produkten**

| | Produkt A (%) | Produkt C (%) |
|---|---|---|
| | 100 | 100 |
| 200 °C / 2h | 94,9 | 93,9 |
| 240 °C / 2h | 91,4 | 86,0 |
| 280 °C / 2h | 89,5 | 82,4 |
| 300 °C /2h | 85,9 | 77,7 |
| 300 °C/ 4h | 82,7 | 76,0 |

Wie aus Tabelle 1 ersichtlich ist, ist das erfindungsgemäßen Produkt **A** wesentlich thermostabiler als der Stand der Technik **Produkt C** (WO-A 2009/015772). Dieses Verhalten war überraschend, da nicht vorhersehbar.

### Beispiel 7: Statische thermische Behandlung der Temper-Produkte B, D und MPP (Melaminpolyphosphat, Stand der Technik)

Die Ergebnisse sind in der Tab. 2. zusammengefasst.

**Tabelle 2: Thermische Behandlung von Temper-Produkten**

| | Produkt B(%) | Produkt D (%) | Produkt MPP (%) |
|---|---|---|---|
| | 100 | 100 | 100 |
| 200 °C / 2h | 99,8 | 99,2 | 99,1 |
| 240 °C / 2h | 99,5 | 98,9 | 98,9 |
| 280 °C / 2h | 98,3 | 97,4 | 98,0 |
| 300 °C /2h | 94,3 | 94,1 | 91,3 |
| 300 °C/ 4h | 89,7 | 92,7 | 83,7 |

Wie aus Tabelle 2 ersichtlich ist, ist das erfindungsgemäße **Produkt B** wesentlich thermostabiler als der Stand der Technik **MPP.** Dieses Verhalten war überraschend, da nicht vorhersehbar.

### Anwendungstechnische Prüfung in PVC

### I. Herstellung der Walzfelle:

Die nach Tabelle 1 (**R-1**, **R-2**) zubereiteten Trockenmischungen werden auf einem Collin-Labormeßwalzwerk (Modell: W100E, BJ: 2005, Fa. COLLIN) jeweils 5 Minuten (Walzendurchmesser: 110 mm, 15 UpM, Friktion: -15 %) bei der angegebenen Temperatur plastifiziert. Die so erhaltenen Folien (Dicke 0,3mm) werden weiteren Messungen zugeführt.

### II. Durchführung des statischen Hitzetestes (SHT):

Von den nach I hergestellten Walzfellen werden Teststreifen (15 mm x 15 mm) ausgeschnitten. Diese werden in einem METRASTAT-Testofen IR 700 (DR. STAPFER GmbH, Düsseldorf) bei der angegebenen Temperatur bis zur signifikanten Verfärbung belastet. Im Anschluss wird der YI-Wert (Yellowness-Index) nach DIN 53381 mit einem Spectro-Guide Farbmessgerät (Fa. BYK-GARDNER) bestimmt und mit dem YI-Wert des unbelasteten Walzfelles verglichen (Nullminutenwert). Die Ergebnisse sind tabellarisch zusammengefasst. Es gilt, je kleiner der YI-Wert zu einem bestimmten Zeitpunkt, desto besser ist das Farbverhalten.

### III. Durchführung der Flammschutzprüfung:

Die oben hergestellten Walzfelle werden zu Pressplatten (120x100x3mm) verarbeitet und einer Flammschutzprüfung in Anlehnung an UL94 zugeführt. Der UL94-Test ist in *"*Flammability of Plastic Materials for Parts in Devices and Appliances", 5th edition, October, 1996 beschrieben.

### IV. Bestimmung der mechanischen Eigenschaften:

Die mechanischen Eigenschaften wurden mittels *Instron 5569* (5kN side action grips) nach ASTM D412 bestimmt.

### V. Durchführung der NMR Messungen:

Alle Messungen wurden auf einem Bruker Avance-II 200 Festkörper-MAS-Spektrometer mit 4.7 T Magnet und einem Doppelresonanzprobenkopf für 2.5 mm Rotoren unter *magic angle spinning* (MAS) Bedingungen durchgeführt. Die verwendeten Rotationsfrequenzen v*_{MAS}* sind bei den entsprechenden Messungen angegeben. Chemische Verschiebungen sind relativ zu den aktuell von der IUPAC empfohlenen Referenzsubstanzen angegeben (²⁷Al: 1.1 M Al(NO₃)₃ in D₂O; ³¹P: 85%ige Phosphorsäure), wobei die Spektrometerkalibrierung mit Hilfe der vereinheitlichten Verschiebungsskala auf die Protonenresonanz von TMS vorgenommen wurde.

Es wurden folgende Formulierungen geprüft:

### Beispiel 8: Prüfung in Weich-PVC:

Folgende Trockenmischungen werden hergestellt (Tabelle 3)- Einwaage in Gew.-Teilen:

**Tab. 3: Formulierungen**

| Komponenten | (**R-1**) | **(R-2)** |
|---|---|---|
| PVC (Evipol SH 7020) K-Wert = 70 | 100 | 100 |
| Weichmacher (DINP)¹⁾ | 50 | 50 |
| Zinkstearat | 0,6 | 0,6 |
| Hydrotalcit²⁾ | 2,9 | 2,9 |
| Antioxidans (Bisphenol A) | 0,5 | 0,5 |
| Flammschtzmittel 1 (ATH)³⁾ | 60 | 25 |
| Flammschutzmittel 2 (Produkt B) | -- | 5 |
| Flammschutzwirkung (Brenndauer in Sek. nach 3 Zündungen) | 0/1/1 | 0/1/1 |

| | | |
|---|---|---|
| ¹⁾ Diisononylphthalat, ex BASF ²⁾ Sorbacid 911, ex SÜD CHEMIE ³⁾ Aluminiumtrihydroxid, APYRAL 40CD, ex NABALTEC | | |

Wie aus Tabelle 3 ersichtlich, schneidet die erfindungsgemäße Formulierung (**R-2**) vergleichbar gut ab, wie der Stand der Technik Beispiel (**R-1**)

**Tabelle 4: SHT (200 °C) nach II**

| Zeit [min] | (**R-1**) | **(R-2)** |
|---|---|---|
| 3 | 10,3 | 5,8 |
| 6 | 10,0 | 5,5 |
| 9 | 11,0 | 5,7 |
| 12 | 11,5 | 6,4 |
| 15 | 12,9 | 7,4 |
| 18 | 13,9 | 8,6 |
| 21 | 15,5 | 10,3 |
| 24 | 18,8 | 12,7 |
| 27 | 22,0 | 15,8 |
| 30 | 25,8 | 19,3 |
| 33 | 31,7 | 24,9 |
| 36 | 40,3 | 33,3 |
| 39 | 52,9 | 44,5 |
| 42 | 70,5 | 62,7 |
| 45 | 95,3 | 76,7 |
| 48 | 110,8 | 85,5 |
| 51 | 117,2 | 89,2 |
| 54 | 118,4 | 90,9 |
| 57 | 117,6 | 91,3 |
| 60 | 115,72 | 92,4 |

Wie aus der Tabelle 4 ersichtlich, zeigt die erfindungsgemäße Formulierung (**R-2**) ein signifikant besseres Farbverhalten, insbesondere in Bezug auf die Anfangsfarbe als die nicht erfindungsgemäße Formulierung (**R-1**)

**Tab. 5.: Mechanische Eigenschaften**

| | Zugfestigkeit [MPa] | Bruchdehnung [%] | Young Modulus [MPa] |
|---|---|---|---|
| (**R-1**) | 13,64 | 331,38 | 33,59 |
| **(R-2)** | 15,24 | 368,45 | 25,27 |

Tabelle 5 zeigt, dass die mechanischen Eigenschaften der erfindungsgemäßen Formulierung (**R-2**) im Vergleich zum Stand der Technik (**R-1**) sogar noch verbessert werden.

## Patentansprüche

1. Eine Flammschutzmittelzusammensetzung, die Folgendes beinhaltet: ein Triazin-interkaliertes Metallphosphat mit mindestens einer Monomereinheit der folgenden allgemeinen Formel (I):
(A-H)a⁽⁺⁾[M_{b}^{m+}(H₂PO₄)ₓ₁⁽⁻⁾(HPO₄)ₓ₂²⁽⁻⁾(PO₄)ₓ₃³⁽⁻)(PO3)_{y}⁽⁻⁾]^{(a-)}∗pH₂O (I)
wobei (A-H)⁽⁺⁾ ein Triazinderivat der folgenden Formel (II-1), (II-2) oder (II-3) ist: 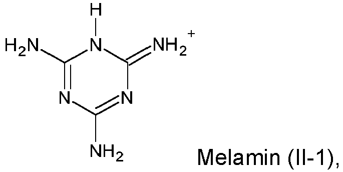 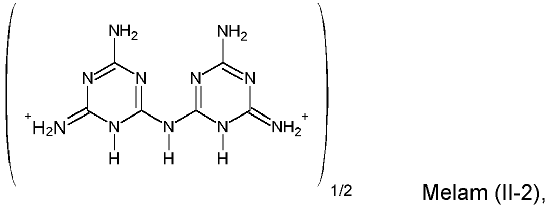 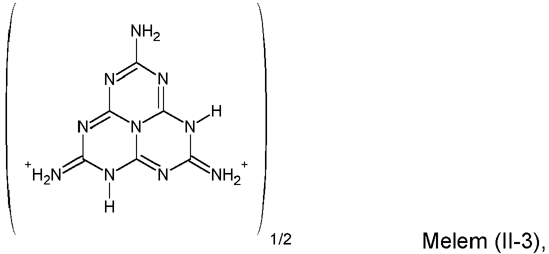 jedes M unabhängig Mg, Zn, oder Al ist;
a 1 bis 6 ist,
b 1 bis 14 ist,
m = 1 bis 4,
x₁, x₂, x₃, y = 0 bis 12, wobei mindestens eine der Variablen x₁, x₂, x₃ > 0 und p = 0 bis 5,
wobei: a + mb = x₁ + 2x₂ + 3x₃ + y; und wobei:
a) das Monomer Folgendes beinhaltet: (i) einen gemischten Typ, zusammengesetzt aus orthophosphat und Pyrophosphatliganden; (ii) einen gemischten Typ, zusammengesetzt aus metaphosphat und Pyrophosphatliganden; oder (iii) Triphosphatliganden beinhaltet; oder
b) wobei x₁ > 0.

2. Triazin-interkaliertes Metallphosphat gemäß Anspruch 1a), wobei y > x₃.

3. Eine Verwendung der Zusammensetzung gemäß Anspruch 1 als Flammschutzmittel in einem Polymer, Papier, Textilien oder Holz-Kunststoff-Verbundwerkstoffen.

4. Verwendung gemäß Anspruch 3, wobei das Polymer thermoplastisch ist, vorzugsweise ausgewählt aus der Gruppe, bestehend aus Polyamid, Polycarbonat, Polyolefin, Polystyrol, Polyester, Polyvinylchlorid, Polyvinylalkohol, ABS und Polyurethan, oder ein Duroplast ist, vorzugsweise ausgewählt aus der Gruppe, bestehend aus Epoxidharz, Phenolharz und Melaminharz.

5. Ein Verfahren zur Herstellung der Triazin-interkalierten Metallphosphate gemäß Anspruch 1, wobei das Verfahren Folgendes beinhaltet: a) direkte Reaktion von wässrigen sauren Metallphosphaten mit Melamin, um einen hydratisierten Vorläufer zu bilden; und b) optionale anschließende thermische Behandlung, um komplexiertes Wasser zu entfernen und ein dehydratisiertes Produkt zu bilden.

## Claims

1. A flame retardant composition comprising: a triazine-intercalated metal phosphate with at least one monomer unit of the following general formula (I):
(A-H)^{a(+)}[M_{b}^{m+}(H₂PO₄)ₓ₁⁽⁻⁾(HPO₄)ₓ₂²⁽⁻⁾(PO₄)ₓ₃³⁽⁻⁾(PO3)_{y}⁽⁻⁾]^{(a-)}∗pH₂O (I)
wherein (A-H)⁽⁺⁾ is a triazine derivative of the following formula (11-1), (II-2) or (II-3): 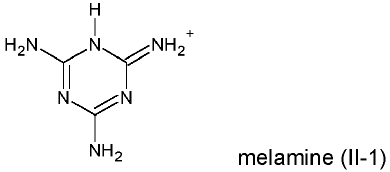 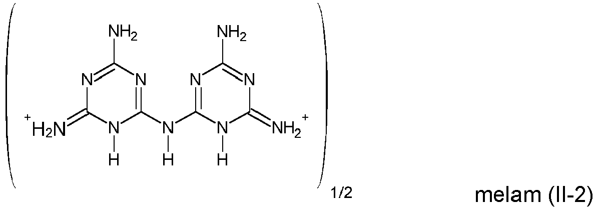 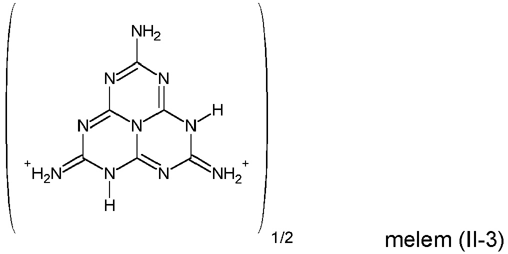 each M is independently Mg, Zn or Al;
a is 1 to 6,
b is 1 to 14,
m = 1 to 4,
x₁, x₂, x₃, y = 0 to 12, wherein at least one of the variables x₁, x₂, x₃ > 0 and p = 0 to 5,
wherein: a + mb = x₁ + 2x₂ + 3x₃ + y; and wherein:
a) the monomer comprises: (i) a mixed type composed of orthophosphate and pyrophosphate ligands; (ii) a mixed type composed of metaphosphate and pyrophosphate ligands; or (iii) triphosphate ligands; or
b) where x₁ > 0.

2. The triazine-intercalated metal phosphate according to claim 1a), wherein y > x₃.

3. Use of the composition of claim 1 as a flame retardant in a polymer, paper, textiles or wood-plastic composites.

4. The use according to claim 3, wherein the polymer is thermoplastic, preferably selected from the group consisting of polyamide, polycarbonate, polyolefin, polystyrene, polyester, polyvinyl chloride, polyvinyl alcohol, ABS and polyurethane, or is a thermoset, preferably selected from the group consisting of epoxy resin, phenol resin and melamine resin.

5. A method for making the triazine-intercalated metal phosphates according to claim 1, wherein the method comprises: a) direct reaction of aqueous acidic metal phosphates with melamine to form a hydrated precursor; and b) optional subsequent thermal treatment to remove complexed water and form a dehydrated product.

## Revendications

1. Une composition d'agent ignifugeant contenant ce qui suit : un phosphate métallique à triazine intercalée avec au moins une unité monomère de la formule générale (I) suivante :
(A-H)ₐ⁽⁺⁾[M_{b}^{m+}(H₂PO₄)ₓ₁⁽⁻⁾(HPO₄)ₓ₂²⁽⁻⁾(PO₄)ₓ₃³⁽⁻⁾(PO₃)_{y}⁽⁻⁾]^{(a-)}∗pH₂O (I)
dans laquelle (A-H)⁽⁺⁾ est un dérivé de la triazine de la formule (II-1), (II-2) ou (II-3) suivante : 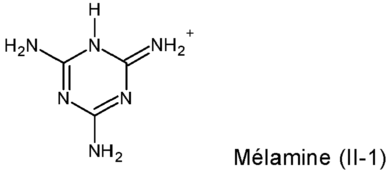 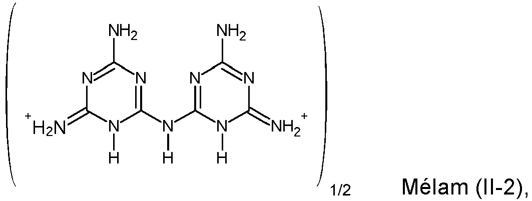 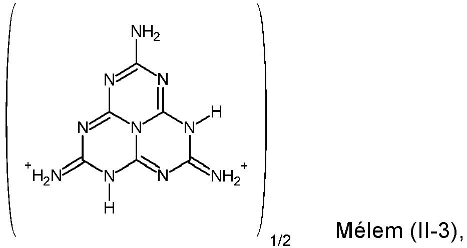 chaque M est indépendamment Mg, Zn, ou Al ;
a va de 1 à 6,
b va de 1 à 14,
m = 1 à 4,
x₁, x₂, x₃, y = 0 à 12, dans laquelle au moins l'une des variables x₁, x₂, x₃ > 0 et p = 0 à 5,
dans laquelle : a + mb = x₁ + 2x₂ + 3x₃ + y ; et dans laquelle :
a) le monomère contient ce qui suit : (i) un type mixte, composé de ligands orthophosphate et pyrophosphate ; (ii) un type mixte, composé de ligands métaphosphate et pyrophosphate ; ou (iii) des ligands triphosphate ; ou
b) dans laquelle x₁ > 0.

2. Phosphate métallique à triazine intercalée selon la revendication 1a), dans lequel y > x₃.

3. Une utilisation de la composition selon la revendication 1 en tant qu'agent ignifugeant dans un polymère, du papier, des textiles ou des matériaux composites bois-matière plastique.

4. Utilisation selon la revendication 3, dans laquelle le polymère est thermoplastique, de préférence sélectionné dans le groupe constitué d'un polyamide, d'un polycarbonate, d'une polyoléfine, d'un polystyrène, d'un polyester, d'un chlorure de polyvinyle, d'un alcool polyvinylique, d'un ABS et d'un polyuréthane, ou est un duroplaste, de préférence sélectionné dans le groupe constitué d'une résine époxyde, d'une résine phénolique et d'une résine de mélamine.

5. Un procédé de production des phosphates métalliques à triazine intercalée selon la revendication 1, le procédé contenant ce qui suit : a) réaction directe de phosphates métalliques acides aqueux avec de la mélamine afin de former un précurseur hydraté ; et b) traitement thermique subséquent facultatif afin de retirer l'eau complexée et de former un produit déshydraté.
